# EUROPEAN PATENT APPLICATION

(11) **EP 4 613 204 A1**
(43) Date of publication of application: **10.09.2025**
(21) Application number: 24161902.2
(22) Date of filing: 07.03.2024
(51) Int. Cl.: A61B 6/58, A61B 6/03, A61B 6/04, G01R 33/48

(54) **MEDICAL SCANNER**

(71) Applicant: Koninklijke Philips N.V., 5656 AG Eindhoven (NL)
(72) Inventor: ENGEL, Klaus Jürgen, Eindhoven (NL); WIEGERT, Jens, Eindhoven (NL); RUETTEN, Walter, 5656AG Eindhoven (NL)
(74) Representative: Philips Intellectual Property & Standards

(57) **Abstract**

A medical imaging scanner has a phantom stored at a phantom storage area. The phantom is moved from the phantom storage area into a scanner bore of the scanner when a scanner calibration is desired. In this way, the phantom becomes part of the scanner and the calibration can be performed remotely or even automatically by the scanner controller.

## Description

### FIELD OF THE INVENTION

This invention relates to medical scanners, and in particular relates to calibration of medical scanners.

### BACKGROUND OF THE INVENTION

In medical imaging, physical phantoms are objects designed to simulate the human body, or parts of it, for specific clinical conditions. Physical phantoms are used to calibrate imaging systems at regular intervals, to evaluate their performance and to ensure the correct operation of imaging systems before scanning human subjects.

For example, in spectral CT, the effective spectrum (i.e., the combination of the actual tube spectrum with the spectral sensitivity of the detector) needs to be determined at regular intervals, as the tube undergoes an aging process, and correspondingly the spectrum of the tube ages. Currently, a system calibration is performed using a phantom containing different materials with different thicknesses.

System calibration is commonly performed by expert service personnel which incurs travelling time and cost, and requires system (phantom) preparation, including downtime of the system during the maintenance service. All of these efforts could be reduced if the calibration process could be (further) automated, for example to enable a fully autonomous calibration or a calibration initiated and monitored by remote service personnel.

### SUMMARY OF THE INVENTION

The invention is defined by the claims.

According to examples in accordance with an aspect of the invention, there is provided a medical imaging scanner, comprising:
a scanner bore;
a patient support;
a phantom storage area;
a phantom which is stored at the phantom storage area at least when not in use; and
a delivery system for moving the phantom from the phantom storage area into the scanner bore when a scanner calibration is desired; and
a calibration system for performing the scanner calibration with the phantom in the scanner bore.

This imaging scanner is able to position a phantom in the scanner bore to enable a calibration procedure. The phantom is part of the overall medical imaging scanner, so that use of the phantom becomes one of the modes of operation of the scanner. This enables the calibration to be controlled remotely or automatically, enabling a remote operator to send instructions to the scanner to apply the phantom and hence perform the calibration.

In one example, the phantom storage area comprises an end portion of the patient support that is movable into the scanner bore, and the delivery system comprises a patient support drive system for moving the patient support. This patient support drive system is the existing drive system of the patient support - it is just controlled with an additional mode of operation which delivers the end portion of the patient support to the scanner bore.

The end portion of the patient support is for example not used during normal scanning, so that it may be considered to be an extension to the patient support specifically for the calibration process.

In another example, the phantom storage area comprises an end portion of the patient support which remains outside the scanner bore, and the delivery system comprises a phantom drive system for moving the phantom along the patient support from the end portion.

In this example, the phantom is stored at the end of the patient support which is not used during normal scanning. The patient support range of movement does not however need to be increased because the phantom is moved along the patient support to a location which is within the scanner bore. The phantom drive system for example comprises a rail system.

In another example, the phantom storage area comprises a base beneath the patient support which in use remains outside the scanner bore, and the delivery system comprises a lifting system for lifting the phantom from the base to the underside of the patient support such that it is positionable in the scanner bore.

In this case, the phantom is stored beneath the patient support (and outside the scanner bore) and is lifted to the patient support when it is to be positioned within the scanner bore. The underside of the patient support for example, comprises a holding system for holding the phantom.

In another example, the phantom storage area comprises a dock remote from the scanner bore and the delivery system comprises a robotic system for transporting the phantom from the dock to the scanner bore. In this way, the phantom storage does not require any adaptation to the design of the other parts of the scanner. The storage area and robotic system can be designed independently of the remainder of the scanner, so this enables a retrofit solution. The robotic system is for example for transporting the phantom from the dock onto the patient support.

In all examples, the scanner may further comprise a monitoring system for monitoring the area in which the scanner is located, and the calibration system is configured to perform calibration taking into account the monitoring.

This enables automated or semi-automated calibration.

The monitoring system for example, comprises a camera and optionally also a motion sensor. The calibration system is for example controllable to perform a calibration autonomously or under remote control.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
- Fig. 1: shows a CT scanner;
- Fig. 2: shows a first example of a phantom storage and delivery system;
- Fig. 3: shows a second example of a phantom storage and delivery system;
- Fig. 4: shows a third example of a phantom storage and delivery system; and
- Fig. 5: shows a first example of a phantom storage and delivery system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a medical imaging scanner that has a phantom stored at a phantom storage area. The phantom is moved from the phantom storage area into a scanner bore of the scanner when a scanner calibration is desired. In this way, the phantom becomes part of the scanner, and the calibration can be performed remotely or even automatically by the scanner controller.

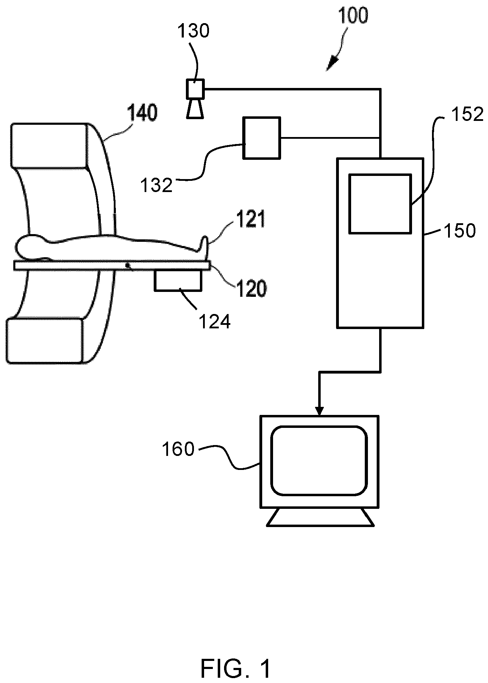

Fig. 1 shows schematically an example of a medical scanner 100 for acquiring a medical image of a subject.

The medical scanner 100 comprises a scanning system such as a CT imaging system 140 that is adapted to acquire a medical image, i.e. a CT image in this example, of a patient 121 positioned on a patient support 120. The patient support 120 is adapted to move the patient 121 through the CT imaging system 140 during the CT imaging procedure. For this purpose, the medical scanner has a drive system 124 for driving the patient support through the scanning system.

Fig. 1 also shows a monitoring system in the form of an optical camera 130 that is adapted to acquire monitoring images of the patient 121 during the CT imaging procedure, and a motion sensor 132. They are discussed below.

A processor 150 performs the processing of the imaging data. In addition, it performs a calibration function, and for this purpose it includes a calibration system 152. The processor 150 generates images for display on a monitor 160.

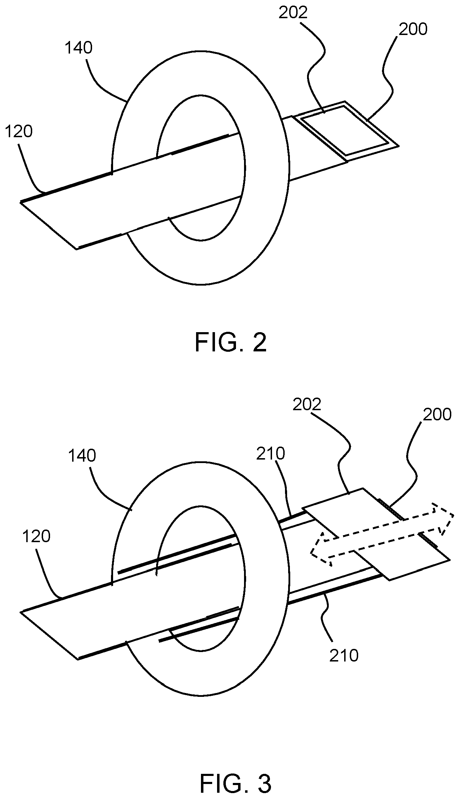

Figs 2 to 5 shows various possible implementations of a phantom storage area and delivery system for moving a phantom from the phantom storage area into the scanner bore when a scanner calibration is desired. By integrating a phantom storage system as part of the scanner, service personnel work can be reduced, and system downtime can be reduced by automating the calibration procedure.

Fig. 2 shows a first example in which a phantom storage area 200 comprises an end portion of the patient support 120 which is movable into the scanner bore. This end portion of the patient support is not used for common CT scans. For example, the length of the patient support can be extended by a few cm, and in the extended length the phantom 202 (comprising filter materials in different combinations) can be statically placed. The delivery system for moving the phantom comprises the already existing patient support drive system 124, and it is controlled to drive the patient support further into the bore than during the conventional CT scans, so that the phantom can be driven into the scanner bore.

Fig. 3 shows a second example in which the phantom storage area 200 again comprises an end portion of the patient support, but in this example the end portion of the patient support remains outside the scanner bore (both during scanning and during calibration). The delivery system comprises a separate phantom drive system for moving the phantom along the patient support from the end portion. Fig. 3 shows a pair of rails 210 along which the phantom 202 is driven so that it can be moved within the scanner bore. A motor is used to drive the phantom along the rails so that it can be translated between the storage area and the position in which calibration will take place.

The patient support range of movement does not need to be increased because the phantom 202 is moved along the patient support to a location which is within the scanner bore.

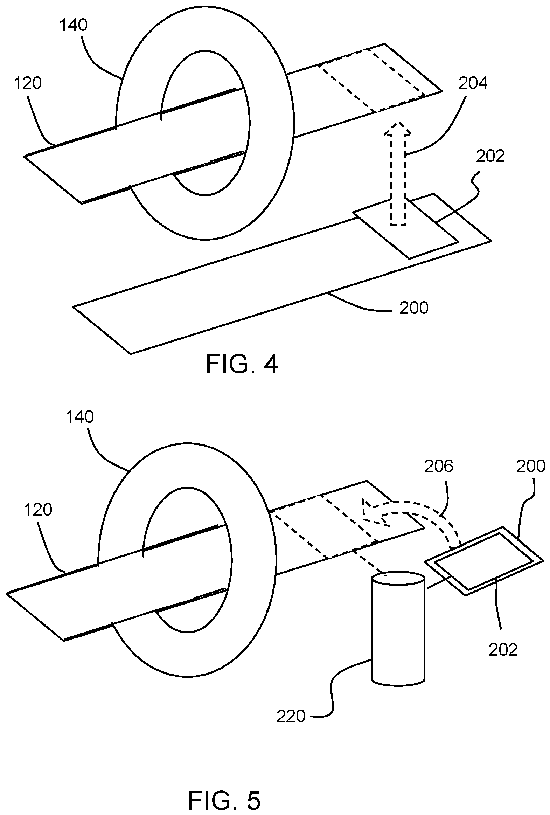

Fig. 4 shows a third example in which the phantom storage area 200 comprises a base beneath the patient support 120 which in use remains outside the scanner bore. The phantom delivery system comprises a lifting system for lifting the phantom from the base to the underside of the patient support 120 (as shown by dotted arrow 204) such that it can then be moved within the scanner bore. The underside of the patient support 120 for example comprises a holding system for holding the phantom. The holding system is for example, a magnetic holder below the patient support.

Fig. 5 shows a third example in which the phantom storage area 200 comprises a dock remote from the scanner bore. A phantom delivery system comprises a robotic system 220 for transporting the phantom from the dock to the scanner bore. For example, the robotic system transports the phantom 202 onto the patient support outside the bore (as shown by dotted arrow 206), and then the patient support drive system moves the phantom, on the patient support, into the scanner bore. The robotic system can also return the phantom from the patient support to the phantom storage area (dock). The robotic system may instead deliver the phantom from the dock directly into the scanner bore independently of the patient support.

The phantom storage does not require any adaptation to the design of the other parts of the scanner. The storage area and robotic system can be designed independently of the remainder of the scanner, enabling a retrofit solution.

A monitoring system is shown in Fig. 1 comprises a camera and motion sensor. The camera enables the CT room to be surveyed by remote service personnel. This allows checking for the absence of local personnel before activating a remote automatic calibration process. Motion sensors for occupancy detection can complement the camera system.

The invention may be applied to any medical scanner in which a calibration using a phantom is desired. Examples are a PET imaging device, an MR imaging device, a SPECT imaging device, as well as a CT scanner as described above. The medical scanner may comprise a C-arm or a closed bore.

Although in the above described embodiments the patient support is always a patient support on which a patient is lying during the acquisition of the medical image, the patient support can also be configured for a sitting or a standing patient.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A medical imaging scanner, comprising:
a scanner bore (140);
a patient support (120);
a phantom storage area (200);
a phantom (202) which is stored at the phantom storage area at least when not in use; and
a delivery system (124; 220) for moving the phantom from the phantom storage area into the scanner bore when a scanner calibration is desired; and
a calibration system (152) for performing the scanner calibration with the phantom in the scanner bore.

2. The scanner of claim 1, wherein the phantom storage area (200) comprises an end portion of the patient support which is movable into the scanner bore, and the delivery system comprises a patient support drive system for moving the patient support.

3. The scanner of claim 1, wherein the phantom storage area (200) comprises an end portion of the patient support which remains outside the scanner bore, and the delivery system comprises a phantom drive system for moving the phantom along the patient support from the end portion.

4. The scanner of claim 3, wherein the phantom drive system comprises a rail system (210).

5. The scanner of claim 1, wherein the phantom storage area (200) comprises a base beneath the patient support which in use remains outside the scanner bore, and the delivery system comprises a lifting system for lifting the phantom from the base to the underside of the patient support such that it is positionable in the scanner bore.

6. The scanner of claim 5, wherein the underside of the patient support comprises a holding system for holding the phantom.

7. The scanner of claim 1, wherein the phantom storage area (200) comprises a dock remote from the scanner bore and the delivery system comprises a robotic system (220) for transporting the phantom from the dock to the scanner bore.

8. The scanner of claim 7, wherein the robotic system is for transporting the phantom from the dock onto the patient support.

9. The scanner of any one of claims 1 to 8, further comprising a monitoring system (130, 132) for monitoring the area in which the scanner is located, wherein the calibration system is configured to perform calibration taking into account the monitoring.

10. The scanner of claim 9, wherein the monitoring system comprises a camera.

11. The scanner of claim 10, wherein the monitoring system further comprises a motion sensor.

12. The scanner of any one of claims 1 to 11, wherein the calibration system is controllable to perform a calibration autonomously or under remote control.
